# EUROPEAN PATENT SPECIFICATION

(11) **EP 1 709 057 B1**
(45) Date of publication and mention of the grant of the patent: **19.12.2007**
(21) Application number: 05701948.1
(22) Date of filing: 19.01.2005
(51) Int. Cl.: C07H 19/16

(54) **SYNTHESIS OF SPONGOSINE**
SYNTHESE VON SPONGOSIN
SYNTHESE DE LA SPONGOSINE

(30) Priority: 21.01.2004 GB 0401292
(43) Date of publication of application: 11.10.2006
(73) Proprietor: Cambridge Biotechnology Ltd, Cambridge CB2 1XJ (GB)
(72) Inventor: OUZMAN, Jacqueline, Valerie, Anne, Cambridge CB2 1XJ (GB)
(74) Representative: Thornton, Neil
(86) International application number: PCT/GB2005/000183
(87) International publication number: WO 2005/070947

(56) References cited:
- BROWN, GEORGE BOSWORTH ET AL: "2- Chloroadenine and 2-chloroadenosine" JOURNAL OF ORGANIC CHEMISTRY ( 1958 ), 23, 125-6 CODEN: JOCEAH; ISSN: 0022-3263, 1958, XP002325338
- BERGMANN W ET AL: "CONTRIBUTIONS TO THE STUDY OF MARINE PRODUCTS. XLIII. THE NUCLEOSIDES OF SPONGES. V. THE SYNTHESIS OF SPONGOSINE" JOURNAL OF ORGANIC CHEMISTRY, AMERICAN CHEMICAL SOCIETY. EASTON, US, vol. 22, December 1957 (1957-12), pages 1575-1577, XP001205635 ISSN: 0022-3263 cited in the application
- BARTLETT R T ET AL: "Synthesis and pharmacological evaluation of a series of analogues of 1-methylisoguanosine" JOURNAL OF MEDICINAL CHEMISTRY, AMERICAN CHEMICAL SOCIETY. WASHINGTON, US, vol. 24, 1981, pages 947-954, XP002225573 ISSN: 0022-2623

## Description

This invention relates to synthesis of spongosine (also known as 9-(β-D-ribofuranosyl)-2-methoxyadenine, or 2-methoxyadenosine) and to synthesis of intermediates for use in the synthesis of spongosine.

The natural product spongosine was first isolated from a sponge, *Cryptotethia crypta,* collected off the Florida coast in 1945 (Bergmann and Feeney, J.Org. Chem. 1951, 16, 981; Ibid 1956, 21, 226). Spongosine was considered an unusual nucleoside in that it was not only the first methoxypurine to be found in nature but also one of the first O-methyl compounds to be isolated from animal tissues.

A method of synthesizing spongosine is described by Bergmann and Stempien (J. Org. Chem. 1957, 22, 1575). This method includes the steps of refluxing chloromercuri-2-methoxyadenine with 2,3,5-tri-*O*-benzoyl-D-ribofuranosyl chloride in xylene to form 9-(2',3',5'-tri-*O*-benzoyl-β-D-ribofuranosyl)-2-methoxyadenine, extracting with chloroform, and refluxing with sodium methoxide in methanol:

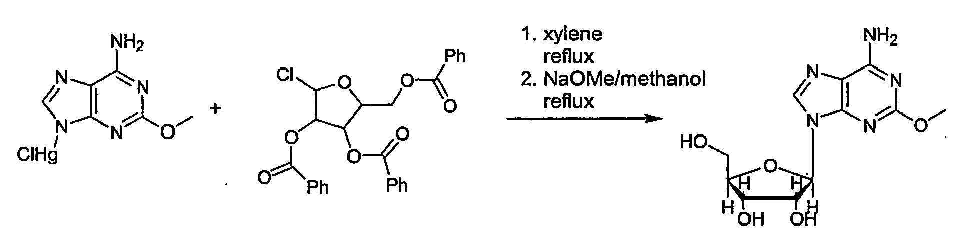

The spongosine produced was obtained in a crude yield of 31 %, and then recrystallised from hot water to provide spongosine which exhibited a melting point of 191-191.5°C and an optical rotation of -43.5° (NaOH).

A disadvantage of this method is that spongosine is not produced in high yield. A further disadvantage is that the method is unlikely to be suitable for large scale synthesis of spongosine because of problems associated with safely removing mercury residues.

Several improvements have been made to this method that allow synthesis of spongosine in high yield and purity, and on a large scale.

It has been found that 9-(2',3',5'-tri-*O*-benzoyl-β-D-ribofuranosyl)-2-methoxyadenine can be obtained by reacting 2-methoxyadenine with 1-*O*-acetyl-2,3,5-tri-*O*-benzoyl-β-D-ribofuranose. This reaction can produce 9-(2',3',5'-tri-*O*-benzoyl-β-D-ribofuranosyl)-2-methoxyadenine in higher yield than the method described by Bergmann and Stempien. Furthermore, the reaction does not produce any mercury residues, and can be carried out at room temperature. This allows the reaction to be more readily scaled-up than the Bergmann and Stempien method.

Thus, according to a first aspect of the invention there is provided a method of synthesizing spongosine, which comprises reacting 1-*O*-acetyl-2,3,5-tri-*O*-benzoyl-β-D-ribofuranose with 2-methoxyadenine to form 9-(2',3',5'-tri-*O*-benzoyl-β-D-ribofuranosyl)-2-methoxyadenine, and deprotecting the 9-(2',3',5'-tri-*O*-benzoyl-β-D-ribofuranosyl)-2-methoxyadenine to form spongosine.

There is also provided according to the invention a method of synthesizing 9-(2',3',5'-tri-*O-*benzoyl-β-D-ribofuranosyl)-2-methoxyadenine, which comprises reacting 1-*O*-acetyl-2,3,5-tri-*O*-benzoyl-β-D-ribofuranose with 2-methoxyadenine.

There is further provided use of 1-*O*-acetyl-2,3,5-tri-*O*-benzoyl-β-D-ribofuranose in the synthesis of spongosine, or 9-(2',3',5'-tri-*O*-benzoyl-β-D-ribofuranosyl)-2-methoxyadenine.

Preferably, reaction of 1-*O*-acetyl-2,3,5-tri-*O*-benzoyl-β-D-ribofuranose with 2-methoxyadenine is carried out by suspending or mixing these reactants in anhydrous acetonitrile and treating the suspension or mixture with trimethylsilyl trifluoromethylsulfonate (TMSOTf), preferably at room temperature.

The 9-(2',3',5'-tri-*O*-benzoyl-β-D-ribofuranosyl)-2-methoxyadenine produced is preferably then isolated. This may be achieved by contacting the reaction mixture with dichloromethane, and preferably washing the mixture (for example with aqueous sodium hydroxide and brine), then precipitating the 9-(2',3',5'-tri-*O*-benzoyl-β-D-ribofuranosyl)-2-methoxyadenine from the organic phase (preferably after drying with magnesium sulfate and filtering). The precipitate is preferably then filtered, washed (for example, with methyl t-butyl ether), and dried.

A preferred method of forming 9-(2',3',5'-tri-*O*-benzoyl-β-D-ribofuranosyl)-2-methoxyadenine is shown in stage 3 of Scheme 2 below. A preferred method of forming and isolating 9-(2',3',5'-tri-*O*-benzoyl-β-D-ribofuranosyl)-2-methoxyadenine is described in the Example below.

Preferably 9-(2',3',5',-tri-*O*-benzoyl-β-D-ribofuranosyl)-2-methoxyadenine is deprotected to form spongosine by treatment with sodium methoxide/methanol.

In the method of Bergmann and Stempien, the product of coupling of chloromercuri-2-methoxyadenine and 2,3,5-tri-*O*-benzoyl-D-ribofuranosyl chloride is treated with sodium methoxide in methanol and refluxed. However, it has been found that the benzoyl groups of 9-(2',3',5',-tri-*O*-benzoyl-β-D-ribofuranosyl)-2-methoxyadenine may be removed by treatment with a methanolic solution of sodium methoxide at ambient temperature. The desired product can be obtained in high yield and purity using this method. In the Example below the purity is 98% (by area LC), and the yield is 86%.

Thus, preferably the 9-(2',3',5',-tri-*O*-benzoyl-β-D-ribofuranosyl)-2-methoxyadenine is deprotected to form spongosine by treatment with sodium methoxide/methanol at room temperature.

According to a second aspect there is provided a method of synthesizing spongosine, which comprises treating 9-(2',3',5',-tri-*O*-benzoyl-β-D-ribofuranosyl)-2-methoxyadenine with sodium methoxide/methanol at room temperature to produce spongosine.

According to the method of Bergmann and Stempien the product of the coupling reaction between chloromercuri-2-methoxyadenine and 2,3,5-tri-*O*-benzoyl-D-ribofuranosyl chloride was isolated, dissolved in anhydrous methanol, and refluxed with a solution of sodium methoxide in methanol. The solution was then dried *in vacuo,* and the semi-solid residue triturated with ether. The remaining crude spongosine was then dissolved in hot water, the solution made slightly basic with sodium hydroxide, and decanted from a brown, oily residue. The extract was then made acidic with hydrochloric acid, and all the liquid removed by freeze-drying. The residual, brown solid was extracted with chloroform, and the extract evaporated to dryness to leave a white residue of spongosine (31% yield).

It has been found that spongosine is obtained in high yield and purity if the spongosine produced by deprotection of 9-(2',3',5',-tri-*O*-benzoyl-β-D-ribofuranosyl)-2-methoxyadenine by treatment with sodium methoxide/methanol is obtained by contacting the spongosine with, or suspending the spongosine in, acetic acid (preferably at room temperature), and then isolating the spongosine.

Thus, preferably the spongosine produced by deprotection of 9-(2',3',5'-tri-*O*-benzoyl-β-D-ribofuranosyl)-2-methoxyadenine is obtained by contacting the spongosine with, or suspending the spongosine in, acetic acid and then isolating the spongosine.

According to a third aspect there is provided a method of synthesizing spongosine, which comprises deprotecting 9-(2',3',5',-tri-*O*-benzoyl-β-D-ribofuranosyl)-2-methoxyadenine to form spongosine, and obtaining the spongosine by contacting the spongosine with, or suspending the spongosine in, acetic acid and then isolating the spongosine.

The spongosine may be isolated by filtration (preferably after concentrating the reaction mixture), washing (for example, with methyl t-butyl ketone), and drying.

A preferred method of deprotecting 9-(2',3',5',-tri-*O*-benzoyl-β-D-ribofuranosyl)-2-methoxyadenine is shown in stage 4 of Scheme 2 below. A preferred method of deprotecting 9-(2',3',5',-tri-*O*-benzoyl-β-D-ribofuranosyl)-2-methoxyadenine and obtaining the spongosine produced is described in the Example below.

It has been found that the yield and purity of spongosine is also improved by dissolving the spongosine in an organic acid, and then crystallizing the dissolved spongosine from the organic acid.

Thus, preferably the spongosine obtained after deprotection of 9-(2',3',5',-tri-*O*-benzoyl-β-D-ribofuranosyl)-2-methoxyadenine is dissolved in an organic acid, and then crystallized from the organic acid.

According to a fourth aspect there is provided a method of synthesizing spongosine, which comprises deprotecting 9-(2',3',5'-tri-*O*-benzoyl-β-D-ribofuranosyl)-2-methoxyadenine to form spongosine, dissolving the spongosine in organic acid, then crystallizing the dissolved spongosine from the organic acid.

Preferably the spongosine is dissolved in the organic acid and crystallized after it has been contacted with, or suspended in, acetic acid and isolated according to a method of the third aspect. However, in some embodiments of the invention, it may be desirable to dissolve the spongosine produced by deprotection of 9-(2',3',5',-tri-*O*-benzoyl-β-D-ribofuranosyl)-2-methoxyadenine in organic acid, and then crystallize the spongosine without having first carried out a method of the third aspect.

Spongosine may be crystallized from the organic acid by contacting the organic acid with an organic alcohol in which spongosine is partially soluble. A preferred organic alcohol is ethanol, although it is believed that isopropanol could alternatively be used. A suitable organic acid is acetic acid.

Spongosine may be dissolved in acetic acid by forming a suspension of spongosine in acetic acid, heating the suspension (for example to 70°C), then if necessary contacting further acetic acid with the heated suspension until a solution is formed. Spongosine may then be crystallized from the solution by contacting the solution with ethanol, and cooling to room temperature.

Crystallised spongosine may be isolated from the acetic acid/organic alcohol using any suitable method. A preferred method is by filtration, washing with methyl t-butyl ketone, then drying.

A preferred method of crystallizing spongosine is shown in stage 5 of Scheme 2 below. A preferred method of dissolving spongosine in acetic acid, and crystallizing then isolating the spongosine is described in the Example below.

According to the method of Bergmann and Stempien, 2-methoxyadenine is formed by heating a mixture of 2-chloroadenine, a solution of sodium in absolute methanol, and a further amount of absolute methanol at 150°C for 5hrs, and then extracting the 2-methoxyadenine produced. However, the yield of 2-methoxyadenine was relatively low (50%).

An improved work up has been developed which comprises adjusting the pH to 9.5 (±0.5) and filtration of the product.

Thus, preferably a mixture of 2-chloroadenine and sodium methoxide/methanol is heated to form 2-methoxyadenine, then the pH of the mixture is adjusted to pH 9.5 (±0.5), and the 2-methoxyadenine then isolated.

According to a fifth aspect there is provided a method of synthesizing spongosine, which comprises heating a mixture of 2-chloroadenine and sodium methoxide/methanol to form 2-methoxyadenine, then adjusting the pH of the mixture to pH 9.5 (±0.5), isolating the 2-methoxyadenine, and forming spongosine from the isolated 2-methoxyadenine.

There is also provided a method of synthesizing 2-methoxyadenine, which comprises heating a mixture of 2-chloroadenine and sodium methoxide/methanol to form 2-methoxyadenine, adjusting the pH of the mixture to pH 9.5 (±0.5), and isolating the 2-methoxyadenine.

Preferably the mixture of 2-chloroadenine and sodium methoxide/methanol is heated to less than 150°C, more preferably to 100°C. Preferably the mixture is cooled and diluted with water. Preferably the pH of the mixture is adjusted to pH 9.5 (±0.5) using hydrochloric acid (preferably the mixture is heated to 60°C). The 2-methoxyadenine may be isolated by filtration, washing (for example, with water then methanol), then drying.

A preferred method of synthesizing 2-methoxyadenine is shown in stage 2 of Scheme 2 below. A preferred method of synthesizing and isolating 2-methoxyadenine is described in the Example below. This method produces 2-methoxyadenine in high yield and is suitable for large scale synthesis (up to 150g).

Spongosine may be formed from the isolated 2-methoxyadenine by any suitable method. Preferably spongosine is formed according to methods of the invention as described above by reacting 2-methoxyadenine with 1-*O*-acetyl-2,3,5-tri-*O*-benzoyl-β-D-ribofuranose to produce 9-(2',3',5'-tri-*O*-benzoyl-β-D-ribofuranosyl)-2-methoxyadenine, then deprotecting the 9-(2',3',5'-tri-*O*-benzoyl-β-D-ribofuranosyl)-2-methoxyadenine to produce spongosine, and preferably then crystallizing the spongosine produced.

There is also provided a method of synthesizing spongosine, which comprises heating 2-chloroadenine with sodium methoxide/methanol at less than 150°C (preferably at 100°C) to form 2-methoxyadenine, and then forming spongosine from the 2-methoxyadenine.

There is further provided a method of synthesizing 2-methoxyadenine, which comprises heating 2-chloroadenine with sodium methoxide/methanol at less than 150°C (preferably at 100°C).

Preferably 2-chloroadenine is synthesized from 2,6-dichloropurine, for example by amination. Amination of 2,6-dichloropurine to 2-chloroadenine is described by Brown and Weliky (J.O.C. 1958 Vol. 23, page 125). 2,6-dichloropurine was heated with 50 volumes of methanol saturated with ammonia at 100°C for 17hrs. Crystals were observed after cooling. The supernatant was evaporated and the residue and crystals dissolved in 1*N* NaOH. The solution was filtered and acidified with acetic acid to give the desired product. However, attempts to repeat this procedure resulted in poor yields due to difficulties in dissolving the residue in 1*N* NaOH. Improved methods of synthesis of 2-chloroadenine have been developed.

There is provided a method of synthesizing 2-chloroadenine, which comprises treating 2,6-dichloropurine with saturated methanolic ammonia to form 2-chloroadenine, diluting with water, and then isolating the 2-chloroadenine produced.

There is also provided according to a sixth aspect a method of synthesizing spongosine, which comprises converting 2,6-dichloropurine to 2-chloroadenine by treating the 2,6-dichloropurine with saturated methanolic ammonia, diluting with water, isolating the 2-chloroadenine produced, and then forming spongosine from the isolated 2-chloroadenine.

Spongosine may be formed from the isolated 2-chloroadenine using any suitable method, but preferably using methods according to the invention.

According to a further aspect there is provided a method of synthesizing 2-methoxyadenine, which comprises converting 2,6-dichloropurine to 2-chloroadenine by treating the 2,6-dichloropurine with saturated methanolic ammonia, diluting with water, isolating the 2-chloroadenine produced, and then forming 2-methoxyadenine from the isolated 2-chloroadenine.

According to a further aspect there is provided a method of synthesizing 9-(2',3',5'-tri-*O-*benzoyl-β-D-ribofuranosyl)-2-methoxyadenine, which comprises converting 2,6-dichloropurine to 2-chloroadenine by treating the 2,6-dichloropurine with saturated methanolic ammonia, diluting with water, isolating the 2-chloroadenine produced, and then forming 9-(2',3',5'-tri-*O*-benzoyl-β-D-ribofuranosyl)-2-methoxyadenine from the isolated 2-chloroadenine.

Preferably the 2,6-dichloropurine and saturated methanolic ammonia are heated, preferably to 100°C. Preferably the 2,6-dichloropurine is treated with less than 50 volumes, preferably 10 volumes, of saturated methanolic ammonia.

A preferred method of synthesizing 2-chloroadenine is shown in stage 1 of Scheme 2 below.

2-chloroadenine may be isolated by filtration, washing (for example with methanol), and drying. A preferred method of synthesizing and isolating 2-chloroadenine is described in the Example below. This procedure was successfully performed on 877g of 2,6-dichloroadenosine, and gave 725g (92 mol%) of product with a purity of 96% (peak area LC).

There is also provided a method of synthesizing spongosine which comprises converting 2,6-dichloropurine to 2-chloroadenine, and then forming spongosine from the 2-chloroadenine.

There is further provided according to the invention use of 2,6-dichloropurine in the synthesis of spongosine.

Scheme 1 below shows consecutive steps in the synthesis of spongosine from 2,6-dichloropurine. The number in brackets after each synthesis step in the scheme corresponds to the particular aspect of the invention that is associated with an improvement to that step.

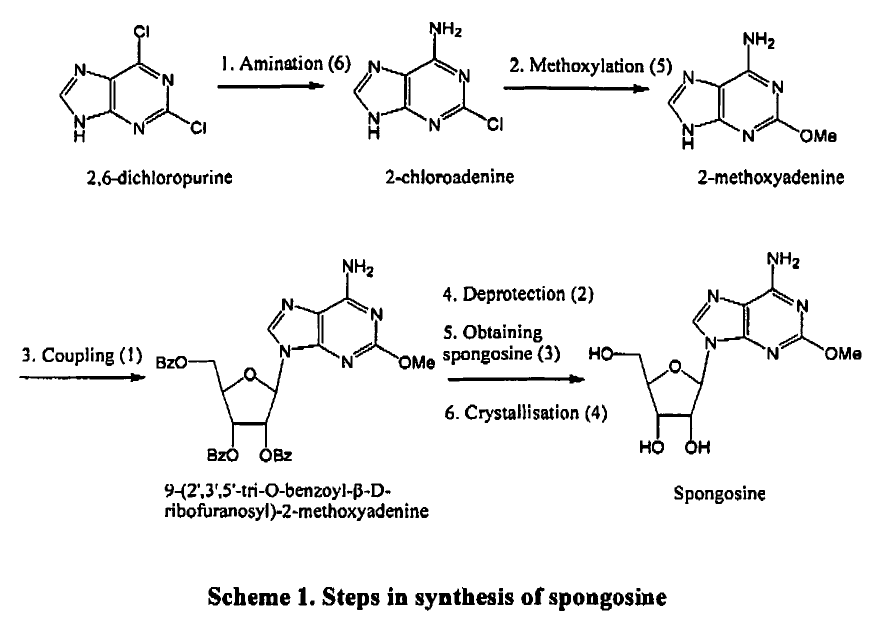

Whilst it is preferred that spongosine is produced by a method that incorporates improvements according to the first aspect of the invention, and the second, third, fourth, fifth, and sixth aspects, it will be appreciated that improvements in the yield of spongosine relative to the method of Bergmann and Stempien may be obtained by use of any one of the different aspects, or by any combination of two or more of the different aspects. If a method does not incorporate all six aspects, the remaining step(s) may be carried out as described by Bergmann and Stempien, or by an alternative method(s).

For example, 9-(2',3',5'-tri-*O*-benzoyl-β-D-ribofuranosyl)-2-methoxyadenine could be formed by a method of the first aspect of the invention, and this protected intermediate then deprotected using the method described in Bergmann and Stempien. The spongosine produced by deprotection could then be obtained by a method of the third aspect, and optionally crystallized by a method of the fourth aspect.

In other embodiments, it may be preferred to carry out synthesis of spongosine starting from 2-chloroadenine, 2-methoxyadenine, or 9-(2',3',5'-tri-*O*-benzoyl-β-D-ribofuranosyl)-2-methoxyadenine, rather than 2,6-dichloropurine. Again, it is preferred that the subsequent steps for the synthesis of spongosine (shown in Scheme 1) are all carried out according to appropriate aspects. However, any one of the different aspects, or any combination of two or more of the different aspects, may be used and the remaining step(s) carried out as described by Bergmann and Stepmien, or by an alternative method(s).

Thus, the different aspects may be used in any of the following combinations:
1^{st} + 2^{nd}; 1^{st} + 3^{rd}; 1^{st} + 4^{th}; 1^{st} + 5^{th}; 1^{st} + 6^{th}; 2^{nd} + 3^{rd}; 2^{nd} + 4^{th}; 2^{nd} + 5^{th}; 2^{nd} + 6^{th}; 3^{rd} + 4^{th}; 3^{rd} + 5^{th}; 3^{rd} + 6^{th}; 4^{th} + 5^{th}; 4^{th} + 6^{th}; or 5^{th} +6^{th};
1^{st} + 2^{nd} + 3^{rd}; 1^{st} + 2^{nd} + 4^{th}; 1^{st} + 2^{nd} + 5^{th}; 1^{st} + 2^{nd} +6^{th}; 1^{st} + 3^{rd} + 4^{th}; 1^{st} + 3^{rd} + 5^{th}; 1^{st} + 3^{rd} + 6^{th}; 1^{st} + 4^{th} + 5^{th}; 1^{st} + 4^{th} +6^{th}; 1^{st} + 5^{th} + 6^{th}; 2^{nd} + 3^{rd} + 4^{th}; 2^{nd} + 3^{rd} + 5^{th}; 2^{nd} + 3^{rd} + 6^{th};
2^{nd} + 4^{th} + 5^{th}; 2^{nd} + 4^{th} + 6^{th}; 2^{nd} + 5^{th} + 6^{th}; 3^{rd} + 4^{th} + 5^{th}; 3^{rd} + 4^{th} + 6^{th}; 3^{rd} + 5^{th} + 6^{th}; or 4^{th} + 5^{th} + 6^{th};
1^{st} + 2^{nd} + 3^{rd} + 4^{th}; 1^{st} + 2^{nd} + 3^{rd} + 5^{th}; 1^{st} + 2^{nd} + 3^{rd} + 6^{th}; 1^{st} + 2^{nd} + 4^{th} + 5^{th}; 1^{st} + 2^{nd} + 4^{th} + 6^{th}; 1^{st} + 2^{nd} + 5^{th} + 6^{th}; 1^{st} + 3^{rd} + 4^{th} + 5^{th}; 1^{st} + 3^{rd} + 4^{th} + 6^{th}; 1^{st} + 3^{rd} + 5^{th} + 6^{th}; 1^{st} + 4^{th} + 5^{th} + 6^{th}; 2^{nd} + 3^{rd} + 4^{th} + 5^{th}; 2^{nd} + 3^{rd} + 4^{th} + 6^{th}; 2^{nd} + 3^{rd} + 5^{th} + 6^{th}; 2^{nd} + 4^{th} + 5^{th} + 6^{th}; or
3^{rd} + 4^{th} + 5^{th} + 6^{th};
1^{st} + 2^{nd} + 3^{rd} + 4^{th} + 5^{th}; 1^{st} + 2^{nd} + 3^{rd} + 4^{th} + 6^{th}; or 1^{st} + 3^{rd} + 4^{th} + 5^{th} + 6^{th}.

Methods of the invention are considerably simpler to carry out than the method of Bergmann and Stempien, provide spongosine in greater yield, and can be used for large scale synthesis of spongosine (or of an intermediate for the synthesis of spongosine).

Embodiments of the invention are now described, by way of example only, with reference to the accompanying Scheme 2 that shows synthesis of spongosine according to a preferred embodiment of the invention.

### Example

### Synthesis of spongosine

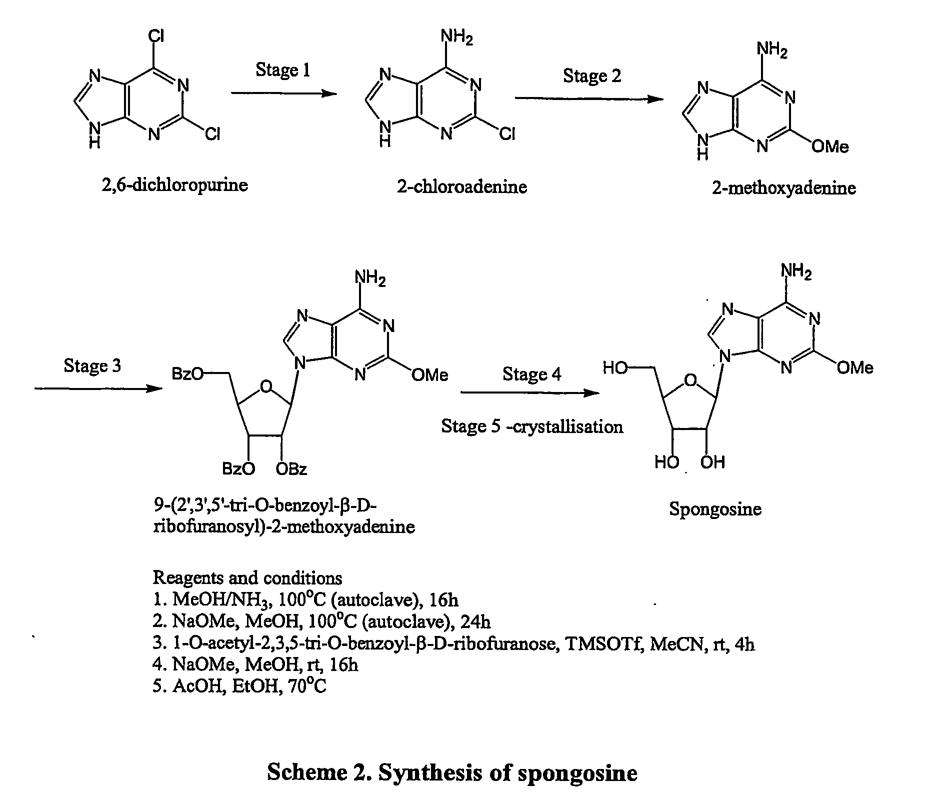

### Preparation of 2-chloroadenine:

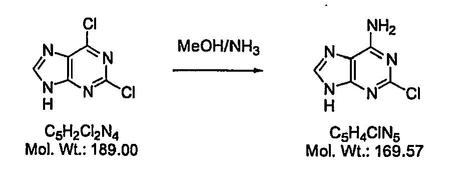

2,6-Dichloropurine (0.877 kg, 4.64 mol) was placed in a 20 L autoclave and treated with methanolic ammonia (7.0N, 6.95 kg). On sealing the autoclave, stirring was initiated and the mixture heated to an internal temperature of 100°C. The reaction mixture was maintained at 100°C for 17 h. before cooling to ambient temperature. Once fully cooled, the autoclave vessel was opened and the reaction mixture diluted with water (3.5 kg). The resulting solid suspension was collected by suction filtration and the filter cake washed with methanol (2 x 0.7 kg) before drying to constant weight in a vacuum oven at 40°C. 2-Chloroadenine was isolated as a pale yellow solid (0.725 kg, 4.28 mol, 92%).
δ_{H} (250 MHz; d₆-DMSO); 7.67 (2H, s, N*H₂*), 8.15 (1H, s, C*H*), 12.40 (1H, br s, N*H*)
LC 96% by peak area

### Preparation of 2-methoxyadenine:

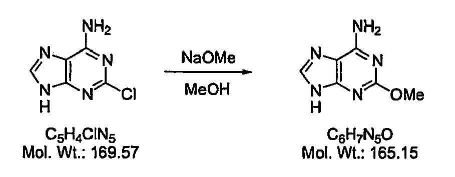

2-Chloroadenine (100 g, 0.59 mmol) was dried by co-evaporation with anhydrous toluene (2 x 200 mL) and then placed in a 2.0L autoclave and treated with sodium methoxide (25% w/w, 1000 ml, 4.63 mol). On sealing the autoclave, stirring was initiated and the mixture heated to an internal temperature of 100°C. The reaction mixture was maintained at 100°C for 24 h. before cooling to ambient temperature. Once fully cooled, the autoclave vessel was opened and the suspension diluted with water (1000 mL). The resulting solution was evaporated under reduced pressure to give a final volume of 1400 mL; to this solution was added water (600mL) to give a final volume of 2000mL. The solution was transferred to a 3.0L, 3-neck flask equipped with an overhead stirrer, pH meter and thermometer. The solution was heated to 60°C (internal temperature) and 50% aq. hydrochloric acid was added at a controlled rate so as to adjust the pH to 9.5 (± 0.5). The resulting suspension was stirred at 60°C for 1 h and cooled slowly to room temperature and stirred for a further 16 h. The suspension was filtered using Whatman No 3 filter paper and the filter cake washed with water (200 mL) and methanol (2 x 200 mL). The solid was dried under vacuum at 50°C to give 2-methoxyadenine (71g, 73 mol%) as an off white solid.
δ_{H} (250 MHz; d₆-DMSO); 3.79 (3H, s, OC*H₃*), 7.18 (2H, br s, N*H₂*), 7.92 (1H, br s, C*H*)
LC 97% by peak area

### Preparation of 9-(2',3',5'-tri-O-benzoyl-β-D-ribofuranosyl)-2-methoxyadenine:

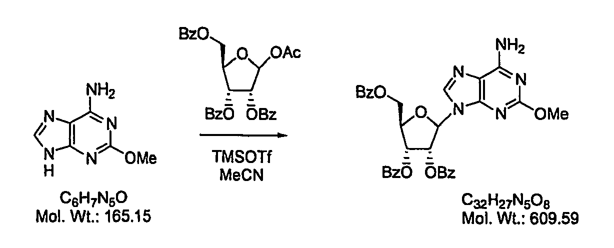

A mixture of 2-methoxyadenine (11 g, 68 mmol) and 1-*O*-acetyl-2,3,5-tri-*O*-benzoyl-β-D-ribofuranose (41 g, 81 mmol) was dried by co-evaporation with anhydrous acetonitrile (2 x 60 mL). To this mixture in anhydrous acetonitrile (328 ml) was added dropwise, trimethylsilyl trifluoromethylsulfonate (29 ml, 36 g, 162 mmol) over 10 minutes (a small exotherm was noted 24-27°C). The suspended material is noted to be gradually solvated during addition. On completion of addition, the reaction mixture was stirred at ambient temperature for 4 h. The reaction mixture was then diluted with dichloromethane (328 mL) and washed with 1N aq. sodium hydroxide (200 mL) and brine (200 mL). The organic phase was dried with magnesium sulfate (20g) and filtered. The solution was then concentrated under reduced pressure to approximately a quarter of its original volume at which point a precipitate started to form. After standing for 1 h the precipitate was filtered and the filter cake washed with methyl t-butyl ether (2 x 100mL). The solid was dried at room temperature to give 9-(2',3',5'-tri-*O*-benzoyl-β-D-ribofuranosyl)-2-methoxyadenine (31g, 75 mol%) as a white solid.
δ_{H} (250 MHz; CDCl₃); 3.92 (3H, s, OC*H₃*), 4.64-4.72 (1H, m, C*H*), 4.79-4.88 (2H, m, 2 x C*H*), 5.79 (2H, br s, N*H₂*), 6.29 (1H, d J3.0, anomeric C*H*), 6.38-6.46 (2H, m, 2 x C*H*), 7.35-7.43 (6H, m, 6 x C*H*), 7.52-7.60 (3H, m, 3 x C*H*), 7.78 (1H, s, C*H*), 7.95-8.00 (6H, m, 6 x C*H*).
LC 97% by peak area

### Preparation of 9-(β-D-ribofuranosyl)-2-methoxyadenine (spongosine):

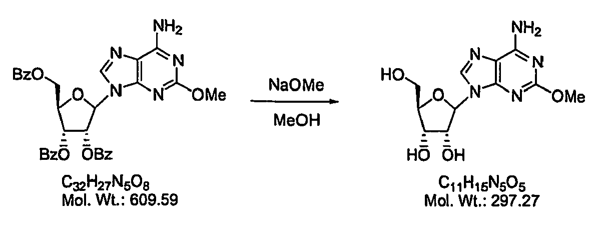

A suspension of 9-(2',3',5'-tri-*O*-benzoyl-β-D-ribofuranosyl)-2-methoxyadenine (39.6 g, 65 mmol) in anhydrous methanol (400 mL) was treated with sodium methoxide (378 mg, 7 mmol, 0.1eq). The mixture was stirred at ambient temperature for 24 h, after which time acetic acid (1g) was added and the suspension stirred for 10 minutes. The mixture was concentrated under reduced pressure to half the volume, and the precipitate filtered. The filter cake was washed with methyl t-butyl ketone (2 x 40 mL) and dried to give 9-(β-D-furanosyl)-2-methoxyadenine (16.7 g 86 mol%) as a white solid.
δ_{H} (250 MHz; CD₃OD); 3.73 (1H, dd J 12.4, 3.4, C*H_{ab}*), 3.86 (1H, dd *J* 12.4, 3.4, C*H_{ab}*), 3.92 (3H, s, OC*H₃*), 4.12 (1H, dd *J* 6.0, 3.0, C*H*), 4.34 (1H. dd *J* 5.0, 3.3, C*H*), 4.75 (1H, t *J* 5.5, C*H*), 5.90 (1H, d *J* 6.0, C*H*), 8.13 (1H, s, C*H*)
δ_{H} (250 MHz; d₆-DMSO); 3.55 (1H, m, C*H*), 3.65 (1H, m, C*H*), 3.83 (3H, s, OC*H₃*), 3.94 (1H, m, C*H*), 4.16 (1H. m, C*H*), 4.63 (1H, m, C*H*), 5.19 (2H, m, O*H*), 5.43 (1H, d *J* 6.1, O*H*), 5.80 (1H, d *J* 6.1, C*H*), 7.35 (2H, bs, N*H*), 8.16 (1H, s, C*H*)
LC greater than 98% by peak area.

### Crystallisation of 9-(β-D-ribofuranosyl)-2-methoxyadenine (spongosine):

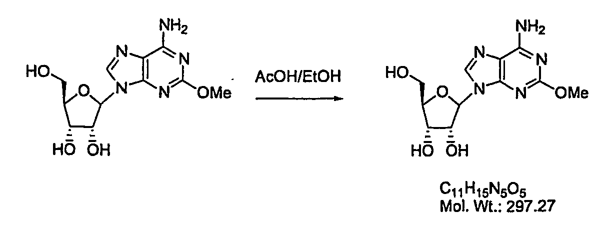

9-(β-D-ribofuranosyl)-2-methoxyadenine (3 g, 10 mmol) was suspended in AcOH (6 mL) and heated to 70°C. A further 6 mL of AcOH was added to give a clear slightly yellow solution; ethanol (48 mL) was added and the solution allowed to cool to room temperature. After standing for 16h the solid was filtered and washed with methyl t-butyl ketone (30 mL). The solid was dried at room temperature to give 9-(β-D-ribofuranosyl)-2-methoxyadenine (2.6g, 86 mol%) as a white solid with a purity greater than 99% and no single impurity greater than 0.5%.

NB: Analysis by ¹H nmr showed the sample to contain residual ethanol (0.24 wt%) and acetic acid (0.29 wt%).

## Claims

1. A method of synthesizing spongosine, which comprises reacting 1-*O-*acetyl-2,3,5-tri-*O*-benzoyl-β-D-ribofuranose with 2-methoxyadenine to form 9-(2',3',5'-tri-*O*-benzoyl- β-D-ribofuranosyl)-2-methoxyadenine, then deprotecting the 9-(2',3',5'-tri-*O*-benzoyl-β-D-ribofuranosyl)-2-methoxyadenine to form spongosine.

2. A method according to claim 1, wherein the 9-(2',3',5-tri-*O*-benzoyl-β-D-ribofuranosyl)-2-methoxyadenine is deprotected by treatment with sodium methoxide/methanol at room temperature.

3. A method according to claim 1 or 2, which further comprises suspending the spongosine in acetic acid, then isolating the spongosine.

4. A method according to claim 3, which further comprises dissolving the isolated spongosine in organic acid, then crystallizing the dissolved spongosine from the organic acid.

5. A method according to claim 1 or 2, which further comprises dissolving the spongosine in organic acid, then crystallizing the dissolved spongosine from the organic acid.

6. A method according to claim 4 or 5, wherein the organic acid is acetic acid.

7. A method according to any of claims 4 to 6, wherein the spongosine is crystallized from the organic acid by contacting the organic acid with an organic alcohol in which spongosine is partially soluble.

8. A method according to any preceding claim, which further comprises heating 2-chloroadenine with sodium methoxide/methanol at less than 150°C, preferably to 100°C, to form the 2-methoxyadenine.

9. A method according to any of claims 1 to 7, which further comprises heating a mixture of 2-chloroadenine and sodium methoxide/methanol to form 2-methoxyadenine, adjusting the pH of the mixture to pH 9.5 (±0.5), and isolating the 2-methoxyadenine before reacting the isolated 2-methoxyadenine with the 1-*O*-acetyl-2,3,5-tri-*O*-benzoyl- β-D-ribofuranose.

10. A method according to claim 9, wherein the mixture is heated to less than 150°C, preferably to 100°C.

11. A method according to any of claims 8-10, which further comprises converting 2,6-dichloropurine to the 2-chloroadenine.

12. A method according to claim 11 in which the 2,6-dichloropurine is converted to 2-chloroadenine by treating the 2,6-dichloropurine with methanolic ammonia to produce 2-chloroadenine, diluting the 2-chloroadenine produced with water, and then isolating the 2-chloroadenine.

13. A method of synthesizing spongosine according to claim 1, which comprises the following steps :

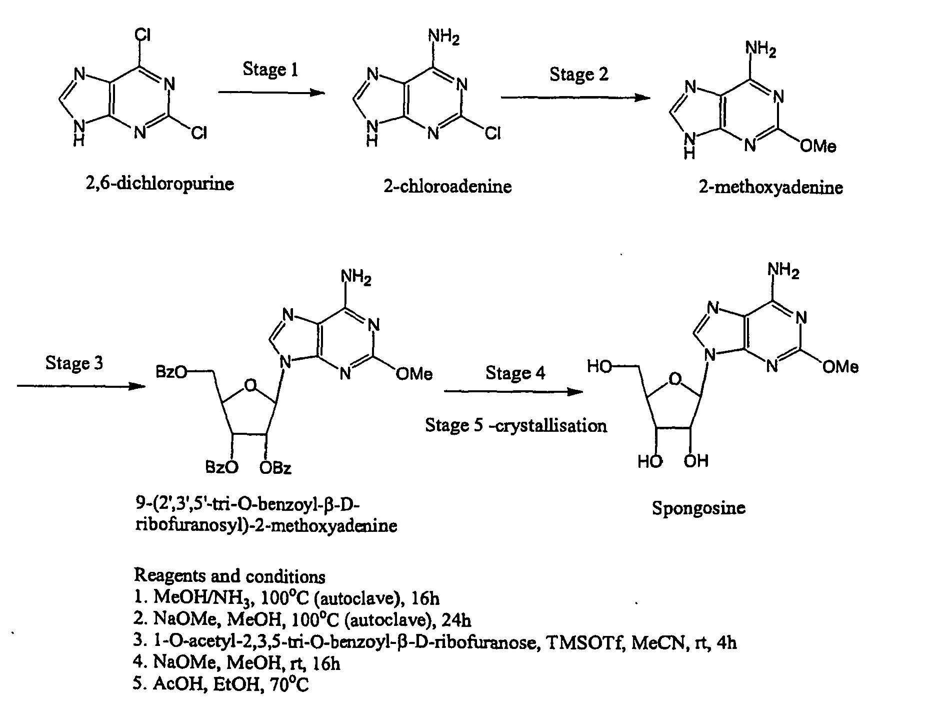

14. A method of synthesizing 9-(2',3',5'-tri-*O*-benzoyl-β-D-ribofuranosyl)-2-methoxyadenine, which comprises reacting 1-*O*-acetyl-2,3,5-tri-*O*-benzoyl-β-D-ribofuranose with 2-methoxyadenine to form 9-(2',3',5'-tri-*O*-benzoyl-β-D-ribofuranosyl)-2-methoxyadenine.

15. Use of 2,6-dichloropurine in the synthesis of spongosine.

## Patentansprüche

1. Verfahren zum Synthetisieren von Spongosin, umfassend das Umsetzen von 1-*O*-Acetyl-2,3,5-tri-O-benzoyl-β-D-ribofuranose mit 2-Methoxyadenin zur Bildung von 9-(2',3',5'-Tri-O-benzoyl-β-D-ribofuranosyl)-2-methoxyadenin, anschließend Entschützen des 9-(2',3',5'-Tri-O-benzoyl-β-D-ribofuranosyl)-2-methoxyadenin zur Bildung von Spongosin.

2. Verfahren gemäß Anspruch 1, wobei das 9-(2',3',5'-Tri-*O*-benzoyl-β-D-ribofuranosyl)-2-methoxyadenin durch Behandlung mit Natriummethoxid/Methanol bei Raumtemperatur entschützt wird.

3. Verfahren gemäß Anspruch 1 oder 2, weiterhin umfassend das Suspendieren des Spongosins in Essigsäure, anschließend Isolieren des Spongosins.

4. Verfahren gemäß Anspruch 3, weiterhin umfassend Lösen des isolierten Spongosins in organischer Säure, anschließend Kristallisieren des gelösten Spongosins aus der organischen Säure.

5. Verfahren gemäß Anspruch 1 oder 2, weiterhin umfassend das Lösen des Spongosins in organischer Säure, anschließend Kristallisieren des gelösten Spongosins aus der organischen Säure.

6. Verfahren gemäß Anspruch 4 oder 5, wobei die organische Säure Essigsäure ist.

7. Verfahren gemäß mindestens einem der Ansprüche 4 bis 6, wobei das Spongosin aus der organischen Säure durch Kontaktieren der organischen Säure mit einem organischen Alkohol, in welchem Spongosin teilweise löslich ist, kristallisiert wird.

8. Verfahren gemäß mindestens einem der vorhergehenden Ansprüche, weiterhin umfassend das Erwärmen von 2-Chloradenin mit Natriummethoxid/Methanol bei weniger als 150ºC, vorzugsweise bis 100ºC, zur Bildung des 2-Methoxyadenins.

9. Verfahren gemäß mindestens einem der Ansprüche 1 bis 7, weiterhin umfassend das Erwärmen einer Mischung von 2-Chloradenin und Natriummethoxid/Methanol zur Bildung von 2-Methoxyadenin, Einstellen des pH-Werts der Mischung auf einen pH-Wert von 9,5 (±0,5) und Isolieren des 2-Methoxyadenin vor dem Umsetzen des isolierten 2-Methoxyadenins mit der 1-O-Acetyl-2,3,5-tri-O-benzoyl-β-D-ribofuranose.

10. Verfahren gemäß Anspruch 9, wobei die Mischung auf weniger als 150ºC, vorzugsweise auf 100ºC erwärmt wird.

11. Verfahren gemäß mindestens einem der vorhergehenden Ansprüche 8-10, weiterhin umfassend das Umwandeln von 2,6-Dichlorpurin zu dem 2-Chloradenin.

12. Verfahren gemäß Anspruch 11, wobei das 2,6-Dichlorpurin zu 2-Chloradenin umgewandelt wird durch Behandeln des 2,6-Dichlorpurins mit methanolischem Ammoniak unter Bildung von 2-Chloradenin, Verdünnen des gebildeten 2-Chloradenins mit Wasser und anschließend Isolieren des 2-Chloradenins.

13. Verfahren zum Synthetisieren von Spongosin gemäß Anspruch 1, umfassend die folgenden Schritte:

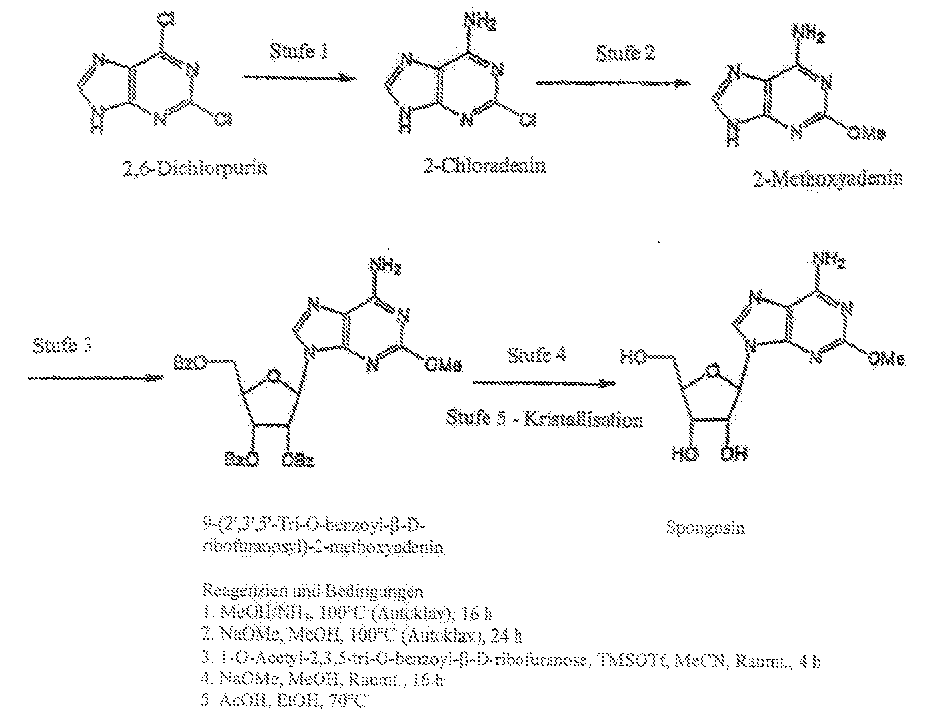

14. Verfahren zum Synthetisieren von 9-(2',3',5'-Tri-O-benzoyl-β-D-ribofuranosyl)-2-methoxyadenin, umfassende das Umsetzen von 1-O-Acetyl-2,3,5-tri-O-benzoyl-β-D-ribofuranose mit 2-Methoxyadenin zur Bildung von 9-(2',3',5'-Tri-O-benzoyl-β-D-ribofuranosyl)-2-methoxy-adenin.

15. Verwendung von 2,6-Dichlorpurin bei der Synthese von Spongosin.

## Revendications

1. Procédé de synthèse de la spongosine, comprenant la réaction de 1-*O*-acétyl-2,3,5-tri-*O*-benzoyl-β-D-ribofuranose avec de la 2-méthoxyadénine pour former de la 9-(2',3',5'-tri-*O*-benzoyl-β-D-ribofuranosyl)-2-méthoxyadénine, puis la déprotection de la 9-(2',3',5'-tri-O-benzoyl-β-D-ribofuranosyl)-2-méthoxyadénine pour former la spongosine.

2. Procédé selon la revendication 1, dans lequel la 9-(2',3',5'-tri-*O*-benzoyl-β-D-ribofuranosyl)-2-méthoxyadénine est déprotégée par un traitement avec du méthoxyde de sodium/méthanol à température ambiante.

3. Procédé selon la revendication 1 ou 2, comprenant en outre la suspension de la spongosine dans l'acide acétique puis l'isolement de la spongosine.

4. Procédé selon la revendication 3, comprenant en outre la dissolution de la spongosine isolée dans un acide organique puis la cristallisation de la spongosine dissoute à partir de l'acide organique.

5. Procédé selon la revendication 1 ou 2, comprenant en outre la dissolution de la spongosine dans un acide organique puis la cristallisation de la spongosine dissoute à partir de l'acide organique.

6. Procédé selon la revendication 4 ou 5, dans lequel l'acide organique est l'acide acétique.

7. Procédé selon l'une quelconque des revendications 4 à 6, dans lequel la spongosine est cristallisée à partir de l'acide organique en mettant en contact l'acide organique avec un alcool organique dans lequel la spongosine est partiellement soluble.

8. Procédé selon l'une quelconque des revendications précédentes, comprenant en outre le chauffage de la 2-chloroadénine avec le méthoxyde de sodium/méthanol à moins de 150 °C, de préférence à 100 ºC, pour former la 2-méthoxyadénine.

9. Procédé selon l'une quelconque des revendications 1 à 7, comprenant en outre le chauffage d'un mélange de 2-chloroadénine et de méthoxyde de sodium/méthanol pour former la 2-méthoxyadénine, l'ajustement du pH du mélange à 9,5 (±0,5) et l'isolement de la 2-méthoxyadénine avant la réaction de la 3-méthoxyadénine isolée avec le 1-*O*-acétyl-2,3,5-tri-*O*-benzoyl-β-D-ribofuranose.

10. Procédé selon la revendication 9, dans lequel le mélange est chauffé à moins de 150 ºC, de préférence à 100 ºC.

11. Procédé selon l'une quelconque des revendications 8 à 10, comprenant en outre la conversion de la 2,6-dichloropurine en 2-chloroadénine.

12. Procédé selon la revendication 11, dans lequel la 2,6-dichloropurine est convertie en 2-chloroadénine en traitant la 2,6-dichloropurine avec de l'ammoniac méthanolique pour produire la 2-chloroadénine, en diluant la 2-chloroadénine produite avec de l'eau et en isolant la 2-chloroadénine.

13. Procédé de synthèse de la spongosine selon la revendication 1, comprenant les étapes suivantes :

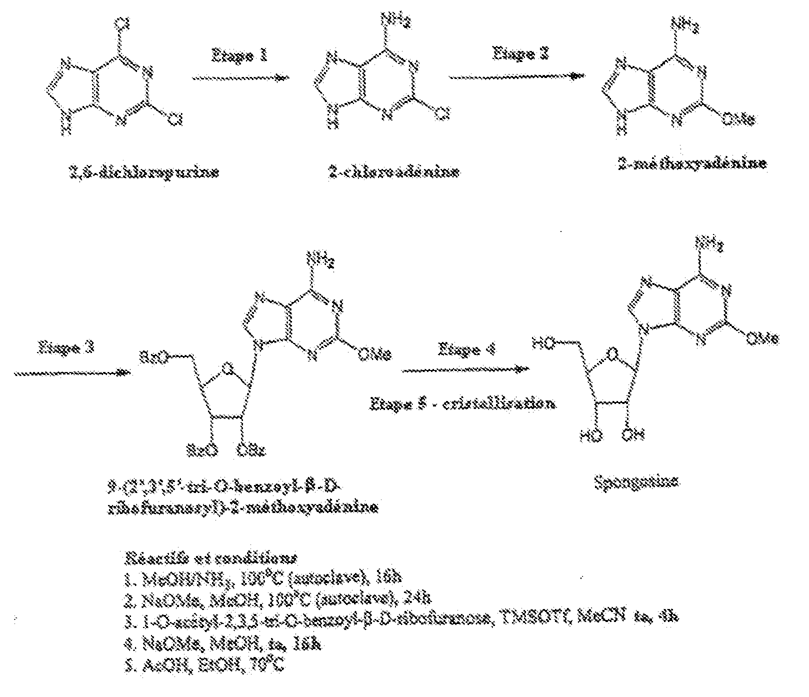

14. Procédé de synthèse de la 9-(2',3',5'-tri-*O*-benzoyl-β-D-ribofuranosyl)-2-méthoxyadénine, comprenant la réaction de 1-*O*-acétyl-2,3,5-tri-*O*-benzoyl-β-D-ribofuranose avec de la 2-méthoxyadénine pour former de la 9-(2',3',5'-tri-*O-*benzoyl-β-D-ribofuranosyl)-2-méthoxyadénine.

15. Utilisation de la 2,6-dichloropurine dans la synthèse de la spongosine.
